(19) Europäisches Patentamt
European Patent Office
Office européen des brevets

(11) Veröffentlichungsnummer: **0 193 813**
**A1**

(12) # EUROPÄISCHE PATENTANMELDUNG

(21) Anmeldenummer: **86102280.4**

(22) Anmeldetag: **21.02.86**

(51) Int. Cl.[4]: **C 07 D 213/30**, C 07 D 213/50, A 01 N 43/40

(30) Priorität: **26.02.85 DE 3506633**

(43) Veröffentlichungstag der Anmeldung: **10.09.86 Patentblatt 86/37**

(84) Benannte Vertragsstaaten: **AT BE CH DE FR GB IT LI NL SE**

(71) Anmelder: **BASF Aktiengesellschaft, Carl-Bosch-Strasse 38, D-6700 Ludwigshafen (DE)**

(72) Erfinder: **Buschmann, Ernst, Dr., Georg-Ludwig-Krebs-Strasse 10, D-6700 Ludwigshafen (DE)**
Erfinder: **Sauter, Hubert, Dr., Neckarpromenade 20, D-6800 Mannheim 1 (DE)**
Erfinder: **Ammermann, Eberhard, Dr., Sachsenstrasse 3, D-6700 Ludwigshafen (DE)**
Erfinder: **Pommer, Ernst-Heinrich, Dr., Berliner Platz 7, D-6703 Limburgerhof (DE)**

(54) **Pyridinderivate und diese enthaltende Fungizide.**

(57) Pyridine der Formel I,

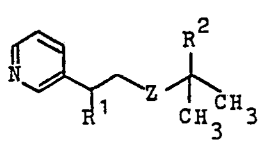

I,

in der
$R^1$ Alkyl, Cycloalkyl, Cycloalkylalkyl, Phenylalkyl, Alkoxyalkyl, Phenoxyalkyl oder Benzyloxyalkyl,
$R^2$ Wasserstoff, Alkoxy, Alkyl, Halogenalkyl, Alkoxyalkyl, Benzyl, Phenyl,
Z C=O, $CR^3OH$ und CHOH bedeuten, wobei $R^3$ Alkyl, Alkenyl, Phenyl oder Benzyl bedeutet,
und die pflanzenphysiologisch verträglichen Säureadditionssalze dieser Verbindung und Fungizide, die diese Verbindungen enthalten.

EP 0 193 813 A1

0193813

Pyridinderivate und diese enthaltende Fungizide

Die vorliegende Erfindung betrifft neue Pyridinderivate und deren Salze, sowie fungizide Mittel, die diese Verbindungen enthalten und Verfahren zu ihrer Herstellung.

Fungizide Pyridinderivate, beispielsweise das S-Butyl-S-(4-tert.-butylbenzyl)-3-pyridylimino-dithiocarbonat werden in der japanischen Patentanmeldung 72/43 334 beschrieben. Ihre fungiziden Wirkungen sind unbefriedigend.

4,4-Dimethyl-1-(4-chlor-phenyl)-1-(3-pyridyl)-3-pentanon ist aus EP 82 058 als Zwischenprodukt bekannt.

Es wurde nun gefunden, daß Pyridine der Formel I

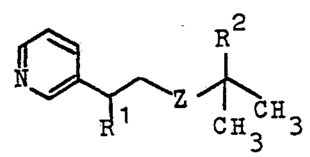 I,

in der $R^1$ für Alkyl, Cycloalkyl, Cycloalkylalkyl, gegebenenfalls substituiertes Phenylalkyl, Alkoxyalkyl, gegebenenfalls substituiertes Phenoxy- und Benzyloxyalkyl, $R^2$ für Wasserstoff, Alkoxy, Alkyl, Halogenalkyl, Alkoxyalkyl, gegebenenfalls substituiertes Benzyl und gegebenenfalls substituiertes Phenyl, Z für C=O, $CR^3OH$ und CHOH stehen, wobei $R^3$Alkyl, Alkenyl und gegebenenfalls substituiertes Phenyl oder Benzyl bedeuten und die pflanzenphysiologisch verträglichen Säureadditionssalze dieser Verbindungen eine gute fungizide Wirkung haben, die der Wirkung der bekannten Pyridinderivate überlegen ist.

$R^1$ bedeutet beispielsweise Alkyl mit 1 bis 12 C-Atomen wie Methyl, Ethyl, n-Propyl, Isopropyl, n-Butyl, Isobutyl, tert.-Butyl, n-Pentyl, Isopentyl, n-Hexyl, n-Heptyl, n-Octyl, n-Dodecyl, Cycloalkyl mit 3 bis 12 C-Atomen wie Cyclopropyl, Cyclopentyl, Cyclohexyl, Cycloheptyl, Cyclooctyl, Cyclododecyl, Cycloalkylalkyl mit 5 bis 6 C-Atomen im Cycloalkylrest und 1 bis 3 C-Atomen im Alkylrest wie Cyclopentylmethyl, Cyclohexylmethyl, 2-Cyclohexyl-ethyl, 3-Cyclohexylpropyl, gegebenenfalls substituiertes Phenylalkyl mit 1 bis 6 C-Atomen im Alkylteil und den Substituenten Halogen, $C_1$-$C_4$-Alkyl, $CF_3$ wie Benzyl, 4-Chlorbenzyl, 2,4-Dichlorbenzyl, 2,3,4-Trichlorbenzyl, 2,6-Dichlorbenzyl, 2,4,5-Trichlorbenzyl, 4-Fluorbenzyl, 4-Brombenzyl, 4-Methylbenzyl, 4-tert.-Butylbenzyl, 4-Methoxybenzyl, 4-$CF_3$-Benzyl, 3-$CF_3$-Benzyl, 2-Phenylethyl, 2-(p-Chlorphenyl)-ethyl, 2-(o-Chlorphenyl)-Sws/Vz

-ethyl, 2-(p-Fluorphenyl)-ethyl, 2-(p-Jodphenyl)ethyl, 2-(p-Methylphenyl)-ethyl, 3-Phenylpropyl, 3-(p-tert.-Butylphenyl)-propyl, 3-(p-Chlorphenyl)-propyl, 3-(p-Fluorphenyl)-propyl, 3-(2,6-Dichlorphenyl)-propyl, 3-Phenyl-2-methyl-propyl, 3-(p-tert.-Butylphenyl)-2-methyl-propyl, 3-(p-Chlorphenyl)-2-methyl-propyl, 3-(p-Methoxyphenyl)-2-methyl-propyl, 3-(p-Methylphenyl)-2-methyl-propyl, 3-(p-Bromphenyl)-2-methyl-propyl, 3-(2,4-Dichlorphenyl)-2-methyl-propyl, 3-(p-Fluorphenyl)-2-methyl-propyl, 3-(o-Fluorphenyl)-2-methyl-propyl, 4-Phenylbutyl, 4-(p-tert.-Butylphenyl)-butyl, 4-(p-Bromphenyl)-butyl, 5-Phenylpentyl, 5-(p-tert.-Butylphenyl)-pentyl, 5-(p-Chlorphenyl)-pentyl, 5-(2,4-Dichlorphenyl)-pentyl, 6-Phenylhexyl, 6-(p-Chlorphenyl)hexyl, gegebenenfalls substituiertes Phenoxyalkyl mit 4 bis 7 C-Atomen im Alkylteil wie 4-Phenoxybutyl, 4-(p-Chlorphenoxy)-butyl, 4-(p-Methoxyphenoxy)-butyl, 4-(2,4-Dichlorphenoxy)-butyl, 4-(p-$CF_3$-Phenoxy)-butyl, 4-(p-Methylphenoxy)-butyl, 5-Phenoxypentyl, 6-Phenoxyhexyl, 7-Phenoxyheptyl, gegebenenfalls substituiertes Benzyloxyalkyl mit 4 bis 7 C-Atomen im Alkylteil und den Substituenten Halogen, $C_1$-$C_4$-Alkyl wie 4-Benzyloxybutyl, 4-(p-Chlorbenzyloxy)-butyl, 4-(p-tert.-Butylbenzyloxy)-butyl,

$R^2$ bedeutet beispielsweise Wasserstoff, Alkoxy mit 1 bis 4 C-Atomen wie Methoxy, Ethoxy, n-Propoxy, Isopropoxy, n-Butoxy, Isobutoxy, Alkyl mit 1 bis 4 C-Atomen wie Methyl, Ethyl, n-Propyl, Isopropyl, n-Butyl, Isobutyl, tert.-Butyl, Halogen-$C_1$-$C_4$-alkyl, Chlormethyl, 2-Chlorethyl, Fluormethyl, Difluormethyl, Benzyl, Halogenbenzyl, 4-Chlorbenzyl, 2,4-Dichlorbenzyl, 4-tert.-Butylbenzyl, 4-Fluorbenzyl, $C_1$-$C_4$-Alkylbenzyl, 4-Methylbenzyl, Phenyl, Halogenphenyl, 4-Chlorphenyl, $C_1$-$C_4$-Alkylphenyl, 4-Methylphenyl.

$R^3$ bedeutet beispielswiese $C_1$-$C_4$-Alkyl, Methyl, Ethyl, n-Propyl, $C_2$-$C_3$-Alkenyl, Vinyl, Allyl, Phenyl, Halogenpenyl, 4-Chlorphenyl, Benzyl, Halogenbenzyl, 4-Chlorbenzyl, 2,4-Dichlorbenzyl.

Pflanzenphysiologisch verträgliche Salze der Verbindungen sind z. B. Hydrobromide, Hydrochloride, Sulfate, Phosphate, Oxalate, Acetate, Formiate oder Additionsverbindungen mit Säuren von Tensiden z. B. Doedecylbenzolsulfonsäure.

Die Verbindungen enthalten mindestens ein asymmetrisches C-Atom. Sie kommen daher in Form ihrer Diastereomere und Enantiomere vor. Die Erfindung umfaßt sowohl die reinen Isomere als auch ihre Mischungen.

Das Verfahren zur Herstellung der neuen Verbindungen ist dadurch gekennzeichnet, daß man ein Pyridylketon der Formel II

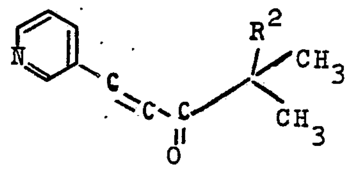 II,

in der $R^2$ die in Anspruch 1 genannten Bedeutungen hat, in Gegenwart eines inerten Lösungsmittels wie Di-n-butylether, Tetrahydrofuran, Dithylether, Dimethoxyethan, Toluol umsetzt mit metallorganischen Verbindungen der Formel $R^1M$ in der M für Li, Na, MgBr und MgCl steht und $R^1$ die in Anspruch 1 genannten Bedeutungen hat. Man erhält so die Pyridylketone der Formel III

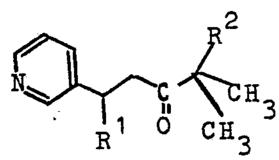 III,

in der $R^1$ und $R^2$ die in Anspruch 1 angegebenen Bedeutungen haben, die gegebenenfalls mit Reduktionsmitteln wie $NaBH_4$, $H_2$/Katalysator, $LiAlH_4$ oder metallorganischen Verbindungen der Formel $R^3M$, in der $R^3$ die in Anspruch 1 genannten Bedeutungen hat und M für Li, Na, MgBr und MgCl steht, zu den Pyridylcarbinolen der Formel IV

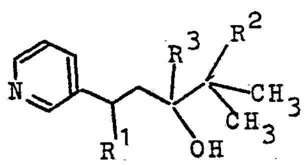 IV,

weiter umgesetzt werden können. Die Alkohole der Formel IV liegen als Diastereomerengemische vor, die mittels Chromatographie oder Kristallisation in die reinen Diastereomere getrennt werden können.

Die folgende Vorschrift und Beispiele erläutern die Herstellung der neuen fungiziden Pyridine:

Vorschrift 1

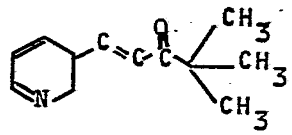

(A)

Zu einer Lösung von 480 ml 10 Gew.-%iger wäßriger NaOH in 240 ml MeOH werden bei 0°C 260 g Pyridin-3-aldehyd zugetropft. Anschließend werden bei 10°C innerhalb 1 h 120 g Pinakolon zugetropft. Man rührt 14 Stunden bei Raumtemperatur (20°C) und extrahiert mit $CH_2Cl_2$. Die organische Phase wird über $Na_2SO_4$ getrocknet, eingeengt und destilliert. Erhalten wurden 140 g (A), Sdp. 124 bis 128°C/0,4 mbar.

Beispiel 1

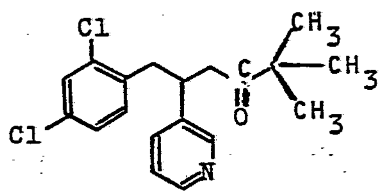

(B)

Zu einer Grignard-Suspension, hergestellt aus 9,8 g 2,4-Dichlorbenzylchlorid, 12 g Mg-Spänen in 50 ml Diethylether werden 7,6 g Vinylketon (A) in 30 ml Diethylether zugetropft. Man erwärmt 2 Stunden zum Rückfluß, hydrolysiert mit gesättigter wäßriger $NH_4Cl$-Lösung. Nach Extraktion mit Diethylether, Trocknen über $Na_2SO_4$ wird eingeengt und destilliert. Erhalten werden 8 g Keton (B), Sdp. 172 bis 176°C/0,2 mbar. Zur weiteren Reinigung wird aus Diethylether umkristallisiert. 3 g Ausbeute, Fp. 100°C (siehe Verbindung Nr. 15).

Beispiel 2

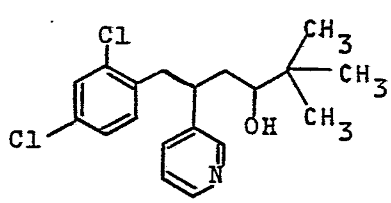

(C)

Zu 5 g Keton (B) in 100 ml MeOH werden 0,4 g $NaBH_4$ zugegeben. Man erwärmt 1 Stunde zum Rückfluß, versetzt mit 200 ml $H_2O$ und extrahiert das Produkt mit $CH_2Cl_2$. Die organische Phase wird über $Na_2SO_4$ getrocknet und eingeengt. Destillation des Rückstandes ergibt 3,6 g (C), farbloses Harz, Sdp. 185°C/0,1 mbar (siehe Verbindung Nr. 16).

Beispiel 3

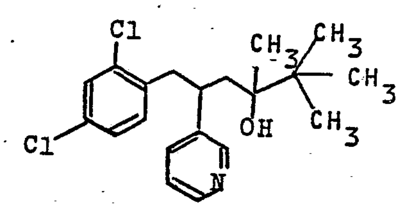

(D)

Zu einer Grignard-Suspension, hergestellt aus 24 g $CH_3J$ und 4 g-Mg-Spänen in 200 ml $Et_2O$ werden 30 g Keton (B) in 250 ml $Et_2O$ zugetropft. Man erwärmt 2 Stunden zum Rückfluß und hydrolysiert mit gesättiger wäßriger $NH_4Cl$-Lösung. Nach Extraktion mit Ether, Trocknen über $Na_2SO_4$ wird eingeengt und destilliert. Man erhält 22 g (D), Sdp. 192°C/0,2 mbar (siehe Verbindung Nr. 19).

In entsprechender Weise werden die folgenden Verbindungen hergestellt.

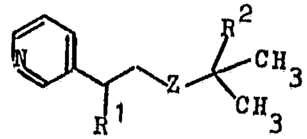

| Nr. | $R^1$ | $R^2$ | Z | Fp. Sdp. °C/mbar |
|---|---|---|---|---|
| 1 | 3-Methylbutyl | $CH_3$ | C=O | |
| 2 | Cyclohexylmethyl | $CH_3$ | C=O | |
| 3 | Cyclohexylmethyl | $CH_3$ | CHOH | |
| 4 | 3-Cyclohexylpropyl | $CH_3$ | C=O | |
| 5 | 3-Cyclohexylpropyl | $CH_3$ | CHOH | |
| 6 | 3-Cyclohexylpropyl | $CH_3$ | $CCH_3OH$ | |
| 7 | Benzyl | $CH_3$ | C=O | 72 - 74 |
| 8 | Benzyl | $CH_3$ | CHOH | |
| 9 | Benzyl | $CH_3$ | $CCH_3OH$ | |
| 10 | 4-Cl-Benzyl | $CH_3$ | C=O | 74 |
| 11 | 4-Cl-Benzyl | $CH_3$ | CHOH | |
| 12 | 4-Cl-Benzyl | $CH_3$ | $CCH_3OH$ | |
| 13 | 4-Brombenzyl | $CH_3$ | C=O | |
| 14 | 4-Brombenzyl | $CH_3$ | CHOH | |
| 15 | 2,4-Dichlorbenzyl | $CH_3$ | C=O | 100 |
| 16 | 2,4-Dichlorbenzyl | $CH_3$ | CHOH | 185/0,1 |
| 17 | 2,4-Dichlorbenzyl | $CH_3$ | CHOH Diastereomer A | 139 |
| 18 | 2,4-Dichlorbenzyl | $CH_3$ | CHOH Diastereomer B | 93 |
| 19 | 2,4-Dichlorbenzyl | $CH_3$ | $CCH_3OH$ | 192/0,2 |
| 20 | 2,4-Dichlorbenzyl | $CH_3$ | C=O Salz mit 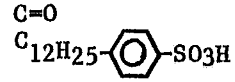 | 117 |

| Nr. | $R^1$ | $R^2$ | Z | Fp.Sdp. °C/mbar |
|---|---|---|---|---|
| 21 | 2,4-Dichlorbenzyl | $CH_3$ | C=O Salz mit HCl | |
| 22 | 2,4-Dichlorbenzyl | $CH_3$ | CHOH Salz mit $C_{12}H_{25}$-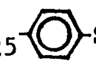-$SO_3H$ | Harz |
| 23 | 2-Phenylethyl | $CH_3$ | C=O | 163/0,1 |
| 24 | 2-Phenylethyl | $CH_3$ | CHOH | |
| 25 | 2-Phenylethyl | $CH_3$ | $CCH_3OH$ | |
| 26 | 2-(p-Chlorphenyl)-ethyl | $CH_3$ | C=O | 202/0,4 |
| 27 | 2-(p-Chlorphenyl)-ethyl | $CH_3$ | CHOH | |
| 28 | 3-Phenylpropyl | $CH_3$ | C=O | 176/0,2 |
| 29 | 3-Phenylpropyl | $CH_3$ | CHOH | |
| 30 | 3-Phenylpropyl | $CH_3$ | $CCH_3OH$ | |
| 31 | 3-(p-Chlorphenyl)-propyl | $CH_3$ | C=O | |
| 32 | 3-(p-Chlorphenyl)-propyl | $CH_3$ | CHOH | |
| 33 | 3-(p-Chlorphenyl)-propyl | $CH_3$ | $CCH_3OH$ | |
| 34 | 2-Methyl-3-phenyl-propyl | $CH_3$ | C=O | 168/0,2 |
| 35 | 2-Methyl-3-phenyl-propyl | $CH_3$ | CHOH | |
| 36 | 2-Methyl-3-phenyl-propyl | $CH_3$ | $CCH_3OH$ | |
| 37 | 2-Methyl-3-(p-methylphenyl)-propyl | $CH_3$ | C=O | 193/0,2 |
| 38 | 2-Methyl-3-(p-methylphenyl)-propyl | $CH_3$ | CHOH | 182/0,4 |
| 39 | 2-Methyl-3-(p-methylphenyl)-propyl | $CH_3$ | $CCH_3OH$ | |
| 40 | 3-(o-Fluorphenyl)-2-methyl-propyl | $CH_3$ | C=O | 180/0,5 |
| 41 | 3-(o-Fluorphenyl)-2-methyl-propyl | $CH_3$ | CHOH | 186/0,4 |
| 42 | 3-(o-Fluorphenyl)-2-methyl-propyl | $CH_3$ | $CCH_3OH$ | |
| 43 | 3-(p-Methoxyphenyl)-2-methyl-propyl | $CH_3$ | C=O | 210/0,4 |
| 44 | 3-(p-Methoxyphenyl)-2-methyl-propyl | $CH_3$ | CHOH | 198/0,2 |
| 45 | 3-(p-Methoxyphenyl)-2-methyl-propyl | $CH_3$ | $CCH_3OH$ | |
| 46 | 3-(p-tert.-Butylphenyl-2-methyl-propyl | $CH_3$ | C=O | 210/0,5 |

| Nr. | $R^1$ | $R^2$ | Z | Fp.Sdp. °C/mbar |
|---|---|---|---|---|
| 47 | 3-(p-tert.-Butylphenyl-2-methyl-propyl | $CH_3$ | CHOH | 215/0,2 |
| 48 | 3-(2,4-$Cl_2$-Phenyl)-2-methyl-propyl | $CH_3$ | C=O | |
| 49 | 3-(2,4-$Cl_2$-Phenyl)-2-methyl-propyl | $CH_3$ | CHOH | |
| 50 | 3-(2,4-$Cl_2$-Phenyl)-2-methyl-propyl | $CH_3$ | $CCH_3OH$ | |
| 51 | 3-(p-Chlorphenyl)-2-methyl-propyl | $CH_3$ | C=O | |
| 52 | 3-(p-Chlorphenyl)-2-methyl-propyl | $CH_3$ | CHOH | |
| 53 | 3-(p-Chlorphenyl)-2-methyl-propyl | $CH_3$ | $CCH_3OH$ | |
| 54 | 4-Phenylbutyl | $CH_3$ | C=O | 185/0,2 |
| 55 | 4-Phenylbutyl | $CH_3$ | CHOH | 190/0,2 |
| 56 | 4-(p-Methylphenyl)-butyl | $CH_3$ | C=O | 211/0,4 |
| 57 | 4-(p-Methylphenyl)-butyl | $CH_3$ | CHOH | |
| 58 | 5-Phenylpentyl | $CH_3$ | C=O | 210/0,1 |
| 59 | 5-Phenylpentyl | $CH_3$ | CHOH | 200/0,2 |
| 60 | 6-Phenylhexyl | $CH_3$ | C=O | 208/0,2 |
| 61 | 6-Phenylhexyl | $CH_3$ | CHOH | 214/0,1 |
| 62 | 4-Phenoxybutyl | $CH_3$ | C=O | |
| 63 | 4-Phenoxybutyl | $CH_3$ | CHOH | 210/0,2 |
| 64 | 4-Phenoxybutyl | $CH_3$ | CHOH Diastereomer A | 83 |
| 65 | 4-Phenoxybutyl | $CH_3$ | CHOH Diastereomer B | 97 |
| 66 | 4-(p-$CH_3$-Phenoxy)-butyl | $CH_3$ | C=O | 200/0,1 |
| 67 | 4-(2,4-Dichlorphenoxy)-butyl | $CH_3$ | C=O | 230/0,2 |
| 68 | 4-(3,5-$Cl_2$-Phenoxy)-butyl | $CH_3$ | C=O | 232/0,3 |
| 69 | 4-(4-Methoxyphenoxy)-butyl | $CH_3$ | C=O | 226/0,2 |
| 70 | 4-(4-Chlorphenoxy)-butyl | $CH_3$ | C=O | 215/0,2 |
| 71 | 5-(4-Chlorphenoxy)-pentyl | $CH_3$ | C=O | 225/0,2 |
| 72 | 6-(4-Chlorphenoxy)-hexyl | $CH_3$ | C=O | 228/0,2 |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora musae an Bananen,
Pseudocercosporella herpotrichoides an Weizen, Gerste,
Pyricularia oryzae an Reis,
Hemileia vastatrix an Kaffee,
Alternaria solani an Kartoffeln, Tomaten
Plasmopara viticola an Reben sowie Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in

bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle, (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz u.a. zur Bekämpfung holzzerstörender Pilze wie Coniophora puteanea und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 10 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 15 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von

40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 15 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 16 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 19 werden den mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 20 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung Nr. 22 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 28 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 46 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd--Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester

2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethylthio-phthalimid

N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Iod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-
-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hyroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-gluataramid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid.
2-Cyano-N-(ethylaminocarbonyl)-2-methoximino)-acetamid
1-(2-(2,4-Dichlorphenyl)-pentyl)-1H-1,2,4-triazol
2,4-Difluor-alpha-(1H-1,2,4-triazolyl-1-methyl)-benzhydrylalkohol

Für die folgenden Versuche wurden als Vergleich die bekannten Verbindungen S-Butyl-S-(4-tert-butyl-benzyl-)-3-pyridyliminodithiocarbonat (A) und 1-(3-Pyridyl)-1-(4-chlorphenyl)-4,4-dimethylpentan-3-on (B) verwendet.

Anwendungsbeispiel 1

Wirksamkeit gegen Gurkenmehltau (kurativ)

Junge Gurkenpflanzen der Sorte "Chinesische Schlagen" werden im Zweiblattstadium mit einer wäßrigen Konidiensuspension des Gurkenmehltaus (Erysiphe cichoracearum und sphaerotheca fuliginea) besprüht. Nach 3 Tagen werden diese Pflanzen mit wäßriger Spritzbrühe, die 80 % Wirkstoff und 20 % Emulgiermittel inder Trockensubstanz enthält, bis zur Tropfnässe besprüht und im Gewächshaus bei Temperaturen zwischen 20 und 22°C und 70 bis 80 % Luftfeuchtigkeit aufgestellt. 21 Tage nach der Wirkstoffapplikation wird das Ausmaß des Pilzbefalls ermittelt.

Das Ergebnis zeigt, daß die Verbindungen 10, 15, 16, 19, 20, 22, 28, 46, 47, 54, 58, 60, 66, 67 bei der Anwendung als 0,0125% und 0,006%ige Spritzbrühen eine bessere fungizide Wirkung zeigten (etwa 97 %) als der bekannte Wirkstoff B (etwa 70 %).

Anwendungsbeispiel 2

Wirksamkeit gegen Weizenbraunrost

Blätter von in Töpfen gewachsenen Weizensämlingen der Sorte "Frühgold" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95 %) gestellt. Während dieser Zeit keimen die Sporen aus und die Keimschläuche dringen indas Blattgewebe ein. Die infizierten Pflanzen werden anschließend mit wäßrigen Spritzbrühen, die 80 % Wirkstoff und 20 % Emulgiermittel in der Trockensubstanz enthalten, tropfnaß gespritz. Nach dem Antrocknen des Spritzbelages werden die Versuchspflanzen im Gewächshaus bei Temperaturen zwischen 20 bis 22°C und 65 bis 70 % relativer Luftfeuchte aufgestellt. Nach 8 Tagen wird das Ausmaß der Rostpilzentwicklung auf den Blättern ermittelt.

Das Ergebnis zeigt, daß die Wirkstoffe 19, 26, 28, 29, 34, 37, 38, 40, 41, 44, 54, 55, 59, 62, 63, 64, 65 bei der Anwendung als 0,025%ige Spritzbrühe eine bessere fungizide Wirkung haben (beispielsweise 97 %) als die bekannten Wirkstoffe A und B (60 %).

Anwendungsbeispiel 3

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis zeigt, daß die Verbindungen 10, 19, 29, 55, 59, 62 eine bessere fungizide Wirkung zeigen (beispielsweise 97 %) als die bekannten Wirkstoffe A (90 %) und B (0 %).

Patentansprüche

1. Pyridine der Formel I

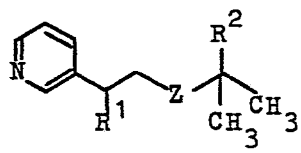 I,

in der

$R^1$ für Alkyl, Cycloalkyl, Cycloalkylalkyl, gegebenenfalls substituiertes Phenylalkyl, Alkoxyalkyl, gegebenenfalls substituiertes Phenoxyalkyl und Benzyloxyalkyl,

$R^2$ für Wasserstoff, Alkoxy, Alkyl, Halogenalkyl, Alkoxyalkyl, gegebenenfalls substituiertes Benzyl, gegebenenfalls substituiertes Phenyl,

Z für C=O, $CR^3OH$ und CHOH stehen, wobei $R^3$ Alkyl, Alkenyl und gegebenenfalls substituiertes Phenyl oder Benzyl bedeuten,

und die pflanzenphysiologisch verträglichen Säureadditionssalze dieser Verbindungen.

2. Pyridine der Formel I gemäß Anspruch 1

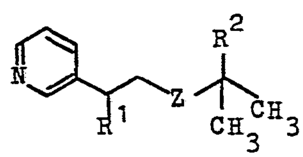 I,

in der $R^1$ die obengenannten Bedeutungen hat, $R^2$ Methyl bedeutet und Z für C=O, $CCH_3OH$ und CHOH steht.

3. Verfahren zur Herstellung eines Pyridins gemäß Anspruch 1, dadurch gekennzeichnet, daß man ein Pyridylketon der Formel II

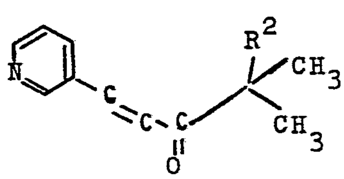 II,

in der $R^2$ die in Anspruch 1 genannten Bedeutungen hat, umsetzt mit einer metallorganischen Verbindung der Formel $R^1M$, in der $R^1$ die in Anspruch 1 genannten Bedeutungen hat und M für Li, Na, MgCl oder MgBr steht, und gegebenenfalls das entstandene Pyridylketon der Formel III,

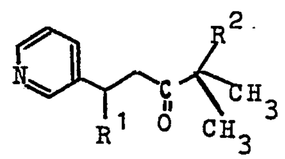
III,

in der $R^1$ und $R^2$ die unter Anspruch 1 genannten Bedeutungen haben, mit $NaBH_4$ oder einer metallorganischen Verbindung $R^3M$ in der $R^3$ die in Anspruch 1 genannten Bedeutungen hat und M für Li, Na, MgCl oder MgBr steht, zum sekundären oder tertiären Carbinol umsetzt.

4. Fungizides Mittel, enthaltend einen inerten Trägerstoff und ein Pyridin der Formel I

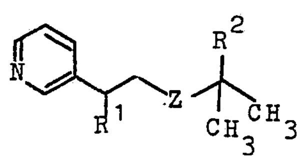
I,

in der

$R^1$ für Alkyl, Cycloalkyl, Cycloalkylalkyl, gegebenenfalls substituiertes Phenylalkyl, Alkoxyalkyl, gegebenenfalls substituiertes Phenoxyalkyl und Benzyloxyalkyl,

$R^2$ für Wasserstoff, Alkoxy, Alkyl, Halogenalkyl, Alkoxyalkyl, gegebenenfalls substituiertes Benzyl, gegebenenfalls substituiertes Phenyl,

Z für C=O, $CR^3OH$ und CHOH stehen, wobei $R^3$ Alkyl, Alkenyl und gegebenenfalls substituiertes Phenyl oder Benzyl bedeuten,

und die pflanzenphysiologisch verträglichen Säureadditionssalze dieser Verbindung.

5. Verfahren zur Bekämpfung von Pilzen, dadurch gekennzeichnet, daß man die Pilze oder die von Pilzbefall bedrohten Materialien, Pflanzen, Böden oder Saatgüter behandelt mit einer fungizid wirksamen Menge eines Pyridins der Formel I

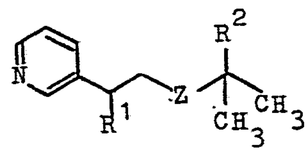

I,

in der

$R^1$ für Alkyl, Cycloalkyl, Cycloalkylalkyl, gegebenenfalls substituiertes Phenylalkyl, Alkoxyalkyl, gegebenenfalls substituiertes Phenoxyalkyl und Benzyloxyalkyl,

$R^2$ für Wasserstoff, Alkoxy, Alkyl, Halogenalkyl, Alkoxyalkyl, gegebenenfalls substituiertes Benzyl, gegebenenfalls substituiertes Phenyl,

Z für C=O, $CR^3OH$ und CHOH stehen, wobei $R^3$ Alkyl, Alkenyl und gegebenenfalls substituiertes Phenyl oder Benzyl bedeuten,

und die pflanzenphysiologisch verträglichen Säureadditionssalze dieser Verbindung.

0193813

Europäisches Patentamt

# EUROPÄISCHER RECHERCHENBERICHT

Nummer der Anmeldung

EP 86 10 2280

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | GB-A-2 015 524 (IMPERIAL CHEMICAL INDUSTIRES LTD.) * Seite 1, Zeile 3 - Seite 2, Zeile 21; Seite 5, Zeilen 5-15 * | 1-5 | C 07 D 213/30<br>C 07 D 213/50<br>A 01 N 43/40 |
| Y,D | EP-A-0 082 058 (UNIVABLOT) * Seite 1, Zeilen 11-16; Seite 2, Formel 2; Seite 3, Zeilen 3-7; Beispiele 1-6 * | 1-5 | |
| A | EP-A-0 016 974 (BAYER AG) * Seite 2, Zeilen 1-18; Seite 3, Zeile 16 - Seite 4, Zeile 18; Seite 5, Zeilen 27, 28; Seite 21, Zeilen 8-20; Seite 22, Zeilen 1-8 * | 1-5 | |
| A | FR-A-2 253 505 (UNICLER) * Seite 1, Zeile 1 - Seite 2, Zeile 5 * | 3 | RECHERCHIERTE SACHGEBIETE (Int. Cl.4)<br>C 07 D 213/00<br>A 01 N 43/00 |

Der vorliegende Recherchenbericht wurde für alle Patentansprüche erstellt.

| Recherchenort | Abschlußdatum der Recherche | Prüfer |
|---|---|---|
| BERLIN | 26-05-1986 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82